(19) Europäisches Patentamt
European Patent Office
Office européen des brevets

(11) Veröffentlichungsnummer: **0 032 561**
**B1**

(12) # EUROPÄISCHE PATENTSCHRIFT

(45) Veröffentlichungstag der Patentschrift: **04.05.83**

(51) Int. Cl.³: **C 07 D 401/12,** A 01 N 43/64 // C07D249/08

(21) Anmeldenummer: **80107906.2**

(22) Anmeldetag: **15.12.80**

(54) **Triazolylphenacyl-pyridyl-ether-Derivate, Verfahren zu ihrer Herstellung sowie ihre Verwendung als Fungizide.**

(30) Priorität: **05.01.80 DE 3000244**

(43) Veröffentlichungstag der Anmeldung: **29.07.81 Patentblatt 81/30**

(45) Bekanntmachung des Hinweises auf die Patenterteilung: **04.05.83 Patentblatt 83/18**

(84) Benannte Vertragsstaaten: **AT BE CH DE FR GB IT LI NL SE**

(56) Entgegenhaltungen:
**EP-A-0 002 678**
**US-A-4 166 854**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Kraatz, Udo, Dr., Körner Strasse 6, D-5090 Leverkusen 1 (DE)**
Erfinder: **Büchel, Karl Heinz, Prof., Dr., Haus an der Dachsdelle Dabringhauser Strasse 42, D-5093 Burscheid (DE)**
Erfinder: **Stetter, Jörg, Dr., Gellertweg 4, D-5600 Wuppertal-1 (DE)**
Erfinder: **Brandes, Wilhelm, Dr., Eichendorffstrasse 3,, D-5653 Leichlingen (DE)**
Erfinder: **Frohberger, Paul-Ernst, Dr., Willi-Baumeister-Strasse 5, D-5090 Leverkusen 1 (DE)**

**EP 0 032 561 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Triazolylphenacyl-pyridyl-ether-Derivate, Verfahren zu ihrer Herstellung sowie ihre Verwendung als Fungizide

Die vorliegende Erfindung betrifft neue Triazolylphenacyl-pyridyl-ether-Derivate, mehrere Verfahren zu ihrer Herstellung sowie ihre Verwendung als Fungizide.

Es ist bereits bekanntgeworden, daß Azolylalkyl-pyridylether-Derivate, wie beispielsweise substituierte 1-Pyridyloxy-3,3-dimethyl-1-triazolyl- bzw. -imidazolyl-butan-2-one bzw. -ole, eine gute fungizide Wirksamkeit besitzen (vergleiche DE-OS-2 756 269 [LeA 18 565]). Deren Wirkung ist jedoch, insbesondere bei niedrigen Aufwandmengen und -konzentrationen, nicht immer ganz befriedigend.

Es wurden neue Triazolylphenacyl-pyridyl-ether-Derivate der Formel

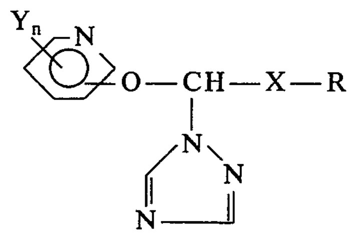

(I)

in welcher

R für Phenyl steht, welches gegebenenfalls substituiert sein kann durch Halogen, Alkyl und Alkoxy mit jeweils 1 bis 4 Kohlenstoffatomen, sowie durch gegebenenfalls durch Halogen substituiertes Phenyl und Phenoxy,
X für die Ketogruppe oder eine CH(OH)-Gruppierung steht,
Y für Halogen, Alkyl oder Alkoxy mit jeweils 1 bis 4 Kohlenstoffatomen oder für Cyano steht und
n für die Zahlen 0, 1, 2, 3 oder 4 steht,

und deren physiologisch verträglichen Säureadditions-Salze sowie Metallsalz-Komplexe gefunden.

Diejenigen Verbindungen der Formel (I), in welchen X für die CH(OH)-Gruppe steht, besitzen zwei asymmetrische Kohlenstoffatome; sie können deshalb in den beiden geometrischen Isomeren (erythro- und threo-Form) vorliegen, die in unterschiedlichen Mengenverhältnissen anfallen können. In beiden Fällen liegen sie als optische Isomeren vor. Sämtliche Isomeren werden erfindungsgemäß beansprucht.

Weiterhin wurde gefunden, daß man die Triazolylphenacylpyridyl-ether-Derivate der Formel (I) erhält, wenn man

a) Triazolylhalogenketone der Formel

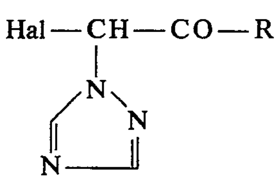

(II)

in welcher

R die oben angegebene Bedeutung hat und
Hal für Chlor oder Brom steht,

mit Pyridinolen der Formel

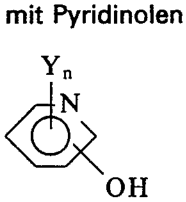

(III)

in welcher

Y und n die oben angegebene Bedeutung haben,

in Gegenwart eines Säurebindemittels und in Gegenwart eines Verdünnungsmittels umsetzt, oder

b) Halogenetherketone der Formel

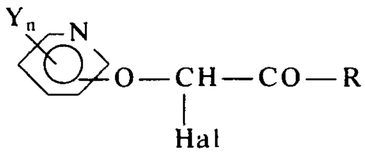

(IV)

in welcher

Hal, R, Y und n die oben angegebene Bedeutung haben,

mit 1,2,4-Triazol in Gegenwart eines Säurebindemittels und gegebenenfalls in Gegenwart eines Verdünnungsmittels umsetzt, und

c) gegebenenfalls die nach den Verfahrensvarianten (a) und (b) erhaltenen Keto-Derivate der Formel

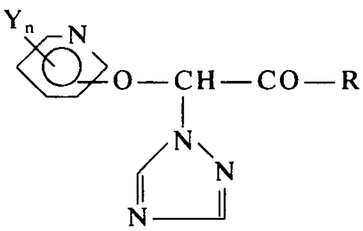

(Ia)

in welcher

R, Y und n die oben angegebene Bedeutung haben,

nach bekannten Methoden in üblicher Weise reduziert.

An die so erhaltenen Verbindungen der Formel (I) kann gegebenenfalls anschließend eine Säure oder ein Metallsalz addiert werden.

Die neuen Triazolylphenacyl-pyridyl-ether-Derivate der Formel (I) weisen starke fungizide Eigenschaften auf. Dabei zeigen überraschenderweise die erfindungsgemäßen Verbindungen eine bessere fungizide Wirksamkeit als die aus dem Stand der Technik bekannten substituierten 1-Pyridyloxy-3,3-dimethyl-1-triazolyl- bzw. -imidazolylbutan-2-one bzw. -ole, welche chemisch und wirkungsmäßig naheliegende Verbindungen sind. Die erfindungsgemäßen Stoffe stellen somit eine Bereicherung der Technik dar.

Die erfindungsgemäßen Triazolylphenacyl-pyridyl-ether-Derivate sind durch die Formel (I) allgemein definiert. Bevorzugt sind diejenigen Verbindungen der Formel (I), in denen R für gegebenenfalls einfach oder zweifach, gleich oder verschieden substituiertes Phenyl steht, wobei als Substituenten genannt seien: Fluor, Chlor, Brom, Methyl, Methoxy, Phenyl, Phenoxy, Chlorphenyl und Chlorphenoxy; Y für Fluor, Chlor, Brom, Jod, Methyl, Methoxy und Cyano steht; X und der Index n die in der Erfindungsdefinition angegebene Bedeutung haben.

Im einzelnen seien außer den bei den Herstellungsbeispielen genannten Verbindungen die folgenden Verbindungen der allgemeinen Formel (I) genannt:

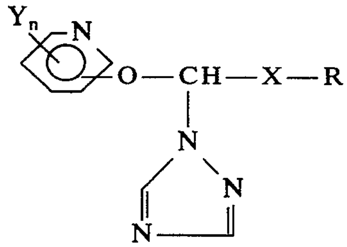

(I)

| 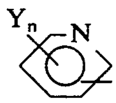 | X | R |
|---|---|---|
| 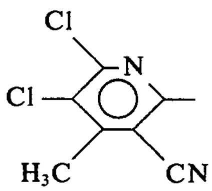 | CO | 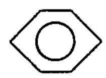 |
| 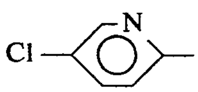 | CO | 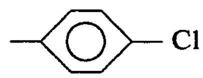 |
| 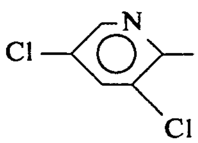 | CO | 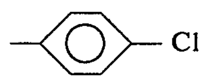 |
| 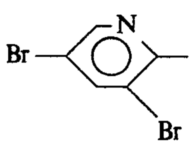 | CO | 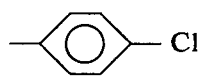 |
| 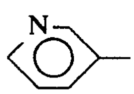 | CO | 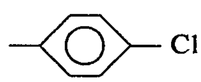 |
| 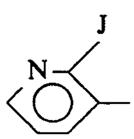 | CO | 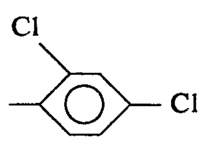 |
| 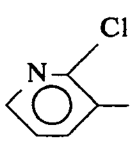 | CO | 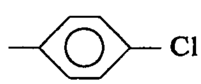 |
| 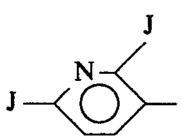 | CO | 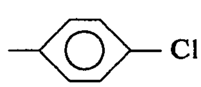 |
| 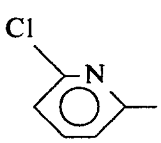 | CH(OH) | 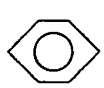 |

Fortsetzung

| 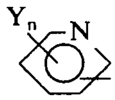 | X | R |
|---|---|---|
| 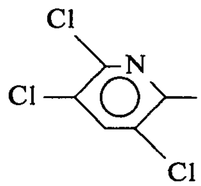 | CH(OH) | 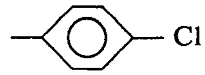 |
| 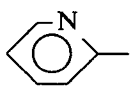 | CH(OH) | 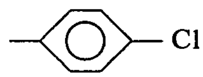 |
| 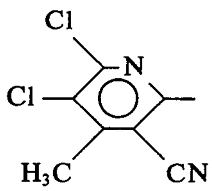 | CH(OH) | 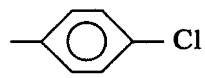 |
| 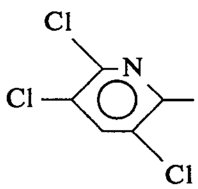 | CH(OH) | 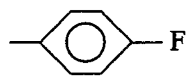 |
| 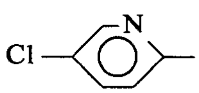 | CH(OH) | 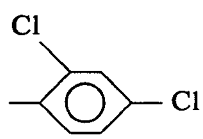 |
| 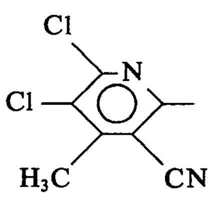 | CH(OH) | 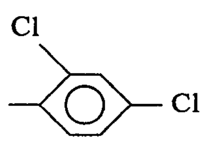 |
| 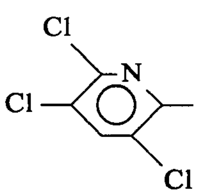 | CH(OH) | 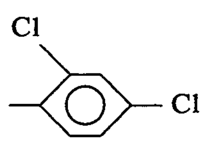 |
| 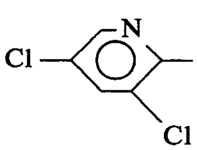 | CH(OH) | 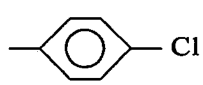 |
| 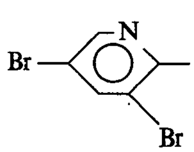 | CH(OH) | 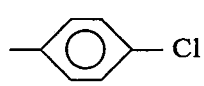 |

Fortsetzung

| 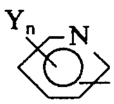 | X | R |
|---|---|---|
| 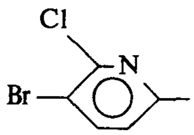 | CH(OH) | 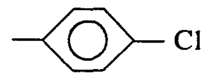 |
| 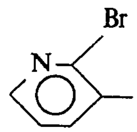 | CH(OH) | 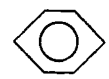 |
| 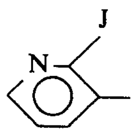 | CH(OH) | 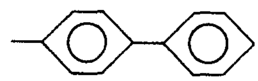 |
| 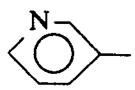 | CH(OH) | 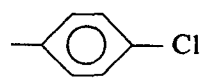 |
| 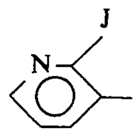 | CH(OH) | 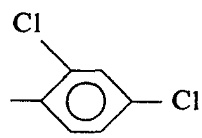 |
| 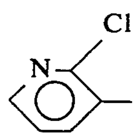 | CH(OH) | 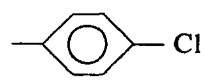 |
| 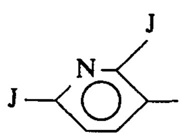 | CH(OH) | 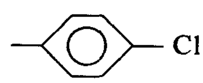 |

Verwendet man beispielsweise ω-Brom-ω-(1,2,4-triazol-1-yl)-4-chloracetophenon und 6-Chlorpyridin-2-ol als Ausgangsstoffe, so kann der Reaktionsablauf durch das folgende Formelschema wiedergegeben werden (Verfahrensvariante a):

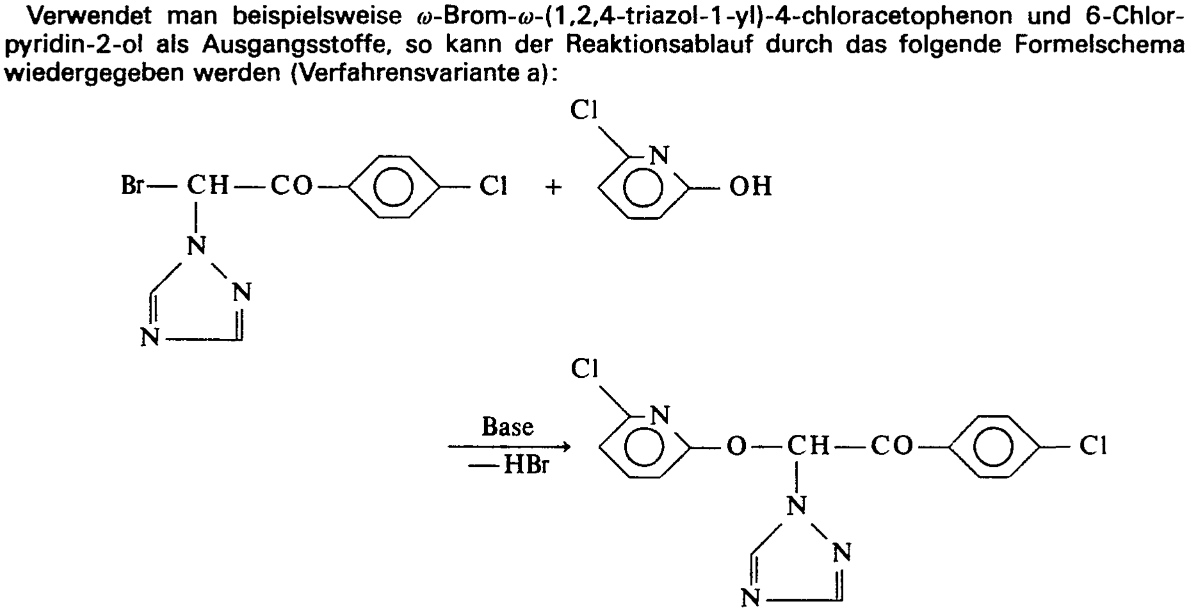

Verwendet man beispielsweise 1-Brom-1-(6-chlor-pyridin-2-yl-oxy)-2-(4-chlorphenyl)-ethan-2-on und 1,2,4-Triazol als Ausgangsstoffe, so kann der Reaktionsablauf durch das folgende Formelschema wiedergegeben werden (Verfahrensvariante b):

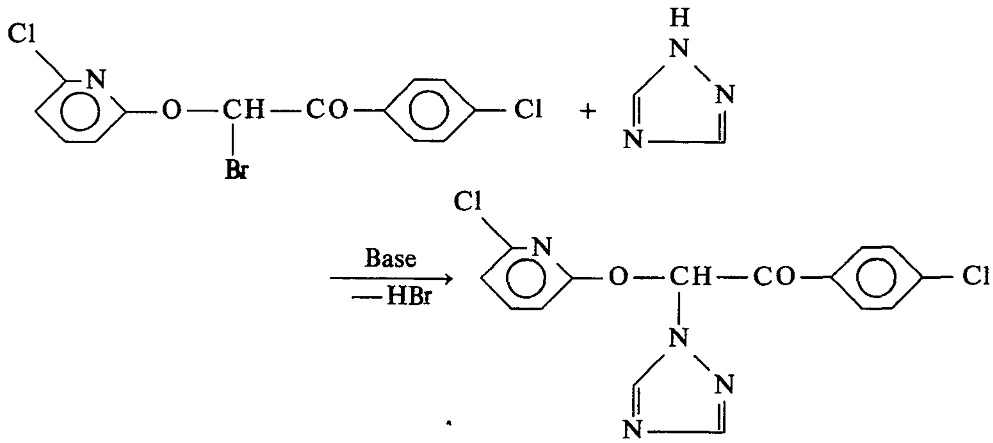


Verwendet man 1-(6-Chlor-pyridin-2-yl-oxy)-2-(4-chlorphenyl)-1-(1,2,4-triazol-1-yl)-ethan-2-on und Natriumborhydrid als Ausgangsstoffe, so kann der Reaktionsablauf durch das folgende Formelschema wiedergegeben werden (Verfahrensvariante c):

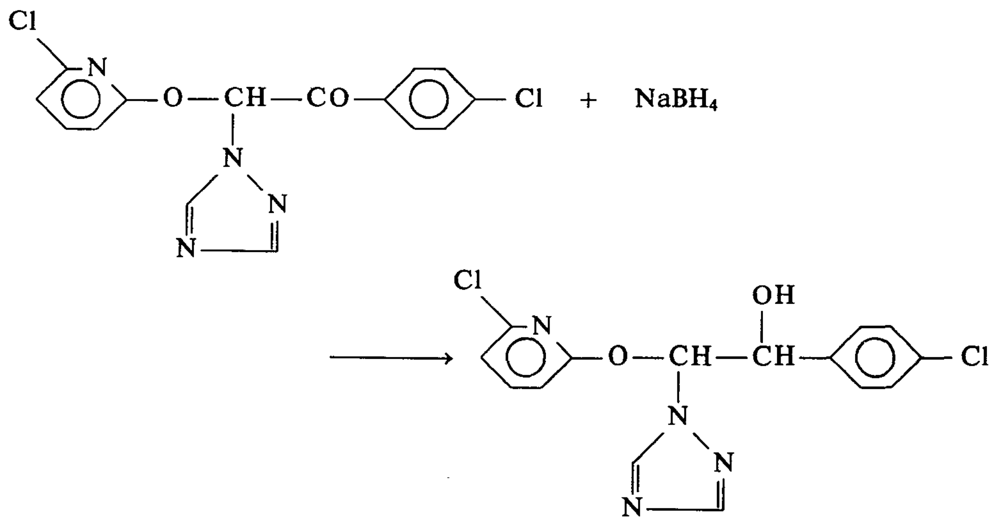

Die als Ausgangsstoffe für die Verfahrensvariante (a) zu verwendenden Triazolylhalogenketone sind durch die Formel (II) allgemein definiert. In dieser Formel steht R vorzugsweise für die Reste, die bereits im Zusammenhang mit der Beschreibung der erfindungsgemäßen Stoffe der Formel (I) vorzugsweise für diesen Substituenten genannt wurden.

Die Triazolylhalogenketone der Formel (II) sind noch nicht bekannt; sie sind jedoch mit Gegenstand einer eigenen älteren Anmeldung, die noch nicht veröffentlicht ist (Deutsche Patentanmeldung P 2 937 595 vom 18. 9. 1979 [LeA 19 934]). Sie werden erhalten, indem man bekannte Triazolylketone (vergleiche DE-OS-2 431 407 [LeA 15 735]) der Formel

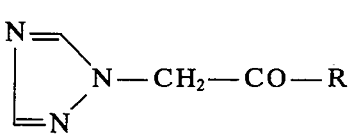
(V)

in welcher

R die oben angegebene Bedeutung hat,

mit Brom oder Chlor in Gegenwart eines sauren Lösungsmittels, wie insbesondere Eisessig, und in Gegenwart eines Halogenwasserstoffakzeptors, wie beispielsweise Natriumhydroxid oder -acetat, bei Temperaturen zwischen 20 und 100°C umsetzt. Die entstehenden Triazolylhalogenketone der Formel

(II) können direkt weiter umgesetzt werden.

Als Ausgangsstoffe der Formel (II) seien beispielsweise genannt:

ω-Brom(Chlor)-ω-(1,2,4-triazol-1-yl)-acetophenon
ω-Brom(Chlor)-ω-(1,2,4-triazol-1-yl)-4-chloracetophenon
ω-Brom(Chlor)-ω-(1,2,4-triazol-1-yl)-4-fluoracetophenon
ω-Brom(Chlor)-ω-(1,2,4-triazol-1-yl)-2-chloracetophenon
ω-Brom(Chlor)-ω-(1,2,4-triazol-1-yl)-2-methylacetophenon
ω-Brom(Chlor)-ω-(1,2,4-triazol-1-yl)-2,4-dichloracetophenon
ω-Brom(Chlor)-ω-(1,2,4-triazol-1-yl)-4-phenylacetophenon
ω-Brom(Chlor)-ω-(1,2,4-triazol-1-yl)-4-(4'-chlorphenylacetophenon)
ω-Brom(Chlor)-ω-(1,2,4-triazol-1-yl)-4-phenoxyacetophenon

Die außerdem für die Verfahrensvariante (a) als Ausgangsstoffe zu verwendenden Pyridinole sind durch die Formel (III) allgemein definiert. In dieser Formel stehen Y und der Index n vorzugsweise für die Reste, die bereits im Zusammenhang mit der Beschreibung der erfindungsgemäßen Stoffe der Formel (I) vorzugsweise für diese Substituenten genannt wurden. Gegebenenfalls werden die Pyridinole der Formel (II) auch in Form ihrer Silbersalze eingesetzt.

Die Pyridinole der Formel (III) sind allgemein bekannte Verbindungen der organischen Chemie. Als Beispiele seien genannt:

2-Hydroxy-pyridin, 3-Hydroxy-pyridin, 4-Hydroxy-pyridin, 2-Hydroxy-6-chlor-pyridin, 3-Hydroxy-5-chlor-pyridin, 2-Hydroxy-4-chlor-pyrdin, 2-Hydroxy-3-chlor-pyridin, 2-Hydroxy-6-brom-pyridin, 2-Hydroxy-5-brom-pyridin, 2-Hydroxy-4-brom-pyridin, 2-Hydroxy-3-brom-pyridin, 2-Hydroxy-6-methyl-pyridin, 2-Hydroxy-5-methyl-pyridin, 2-Hydroxy-4-methylpyridin, 2-Hydroxy-3-methyl-pyridin, 2-Hydroxy-6-fluor-pyridin, 2-Hydroxy-5-fluor-pyridin, 2-Hydroxy-4-fluor-pyridin, 2-Hydroxy-3-fluor-pyridin, 3-Hydroxy-2-chlor-pyridin, 3-Hydroxy-2-brom-pyridin, 3-Hydroxy-2-fluor-pyridin, 3-Hydroxy-2-jod-pyridin, 3-Hydroxy-2-methoxy-pyridin, 3-Hydroxy-6-chlor-pyridin, 3-Hydroxy-5-chlor-pyridin, 4-Hydroxy-2-chlor-pyridin, 4-Hydroxy-pyridin, 4-Hydroxy-3-chlor-pyridin, 2-Hydroxy-3,5,6-trichlorpyridin, 2-Hydroxy-3-cyano-5,6-dichlor-4-methyl-pyridin, 2-Hydroxy-5-brom-6-chlor-pyridin, 2-Hydroxy-5-chlor-4,6-dimethyl-3-cyano-pyridin, 2-Hydroxy-3,5-dichlor-pyridin, 2-Hydroxy-5-brom-4,6-dimethyl-3-cyano-pyridin, 3-Hydroxy-2,6-dijodpyridin, 2-Hydroxy-3,5-dibrom-6-chlorpyridin, 2-Hydroxy-5-brom-6-chlor-3-cyano-4-methylpyridin.

Die für die Verfahrensvariante (b) als Ausgangsstoffe zu verwendenden Halogenetherketone sind durch die Formel (IV) allgemein definiert. In dieser Formel stehen R, Y und der Index n vorzugsweise für die Reste, die bereits im Zusammenhang mit der Beschreibung der erfindungsgemäßen Stoffe der Formel (I) vorzugsweise für diese Substituenten genannt wurden.

Die Halogenetherketone der Formel (IV) sind noch nicht bekannt, können aber nach bekannten Verfahren hergestellt werden, indem man Pyridinole der Formel (III) mit bekannten Halogenketonen (vergleiche DE-OS-2 431 407 [LeA 15 735]) der Formel

$$Hal-CH_2-CO-R \qquad (VI)$$

in welcher

Hal und R die oben angegebene Bedeutung haben,

in Gegenwart eines Säurebindemittels, wie z. B. Kaliumcarbonat, und in Gegenwart eines inerten organischen Lösungsmittels, wie z. B. Aceton, bei Temperaturen zwischen 60 und 120° C umsetzt. Eines der beiden aktiven Wasserstoffatome wird anschließend in üblicher Weise gegen Chlor oder Brom ausgetauscht.

Für die erfindungsgemäßen Umsetzungen gemäß Verfahrensvarianten (a) und (b) kommen als Verdünnungsmittel inerte organische Lösungsmittel in Frage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Formamide; wie insbesondere Dimethylformamid; und halogenierte Kohlenwasserstoffe.

Die Umsetzungen nach Verfahren (a) und (b) werden in Gegenwart eines Säurebinders vorgenomen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat oder wie Silbercarbonat, oder wie niedere tertiäre Alkylamine, Cycloalkylamine oder Aralkylamine, beispielsweise Triethylamin, Dimethylbenzylamin; oder wie Pyridin und Diazabicyclooctan.

Bei der Verfahrensvariante (b) kann auch ein entsprechender Überschuß an Azol verwendet werden.

Die Reaktionstemperaturen können bei den Verfahren (a) und (b) in einem größeren Bereich variiert werden. Im allgemeinen arbeitet man zwischen etwa 20 bis etwa 150° C, vorzugsweise bei 60 bis 120° C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (a) bzw. (b) setzt man auf 1 Mol der Verbindungen der Formel (II) bzw. (IV) vorzugsweise 1 bis 2 Mol Pyridinol der Formel (III) bzw. 1 bis 2 Mol Azol und jeweils 1 bis 2 Mol Säurebinder ein. Zur Isolierung der Verbindungen der Formel (I) wird das Lösungsmittel abdestilliert und der Rückstand entweder mit Wasser versetzt und kräftig gerührt, wobei das Reaktionsprodukt durchkristallisiert, oder mit einem Gemisch aus einem organischen Solvens und Wasser aufgenommen, die organische Phase abgetrennt, mit Wasser gewaschen, über Natriumsulfat getrocknet und im Vakuum vom Lösungsmittel befreit. Der Rückstand wird gegebenenfalls durch Destillation oder Umkristallisation gereinigt.

Die erfindungsgemäße Reduktion gemäß Verfahrensvariante (c) erfolgt in üblicher Weise, z. B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30° C, vorzugsweise bei 0 bis 20° C, durchgeführt. Hierzu setzt man auf 1 Mol des Ketons der Formel (Ia) etwa 1 Mol eines komplexen Hydrids, wie Natriumhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol oder inerte Kohlenwasserstoffe, wie Benzol, in Frage. Die Reaktionstemperaturen können wiederum in einem größeren Bereich variiert werden; im allgemeinen arbeitet man zwischen 20 und 120° C, vorzugsweise bei 50 bis 100° C. Zur Durchführung der Reaktion setzt man auf 1 Mol des Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt und die entstandene Aluminium-Verbindung mit verdünnter Schwefelsäure oder Natronlauge zersetzt. Die weitere Aufarbeitung erfolgt in üblicher Weise.

Zur Herstellung von physiologisch verträglichen Säureadditionssalzen der Verbindungen der Formel (I) kommen vorzugsweise folgende Säuren in Frage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie Getreidemehltau und Gerstenmehltau, von Venturia-Arten, wie gegen den Erreger des Apfelschorfs (Fusicladium dendriticum), und von Podosphaera-Arten, wie gegen den Erreger des echten Apfelmehltaus (Podosphaera leucotricha), eingesetzt werden.

Besonders hervorzuheben ist, daß die erfindungsgemäßen Wirkstoffe nicht nur eine protektive Wirkung haben, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

## Herstellungsbeispiele

### Beispiel 1

(Verfahrensvariante a)

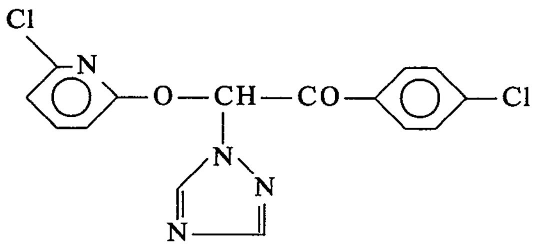

30 g (0,1 Mol) rohes ω-Brom-ω-(1,2,4-triazol-1-yl)-4-chloracetophenon werden in 50 ml Acetonitril gelöst und unter Rühren zu 13 g (0,1 Mol) 6-Chlor-2-hydroxy-pyridin und 10,5 kg (0,01 Mol) Triethylamin in 120 ml Acetonitril gegeben. Man erhitzt 1 Stunde unter Rückfluß und engt anschließend durch Abdestillieren des Lösungsmittels im Vakuum ein. Der Rückstand kristallisiert nach Verrühren mit Wasser. Nach Umkristallisation aus Ethanol erhält man 23 g (66% der Theorie) 1-(6-Chlorpyridin-2-yl-oxy)-2-(4-chlorphenyl)-1-(1,2,4-triazol-1-yl)-ethan-2-on vom Schmelzpunkt 162° C.

### Herstellung des Vorproduktes

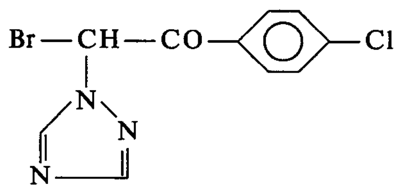

22,1 g (0,1 Mol) ω-(1,2,4-Triazol-1-yl)-4-chloracetophenon werden in 150 ml Eisessig gelöst und nach Zusatz von 8,2 g (0,1 Mol) Natriumacetat bei 45° C tropfenweise mit 16 g (0,1 Mol) Brom bis zur völligen Entfärbung versetzt. Danach gießt man die Reaktionsmischung auf Eiswasser und extrahiert mit Chloroform. Die organische Phase wird mit Natriumhydrogencarbonatlösung gewaschen, über Magnesiumsulfat getrocknet und eingeengt. Man erhält nahezu quantitativ rohes ω-Brom-ω-(1,2,4-triazol-1-yl)-4-chloracetophenon, das direkt weiter umgesetzt wird.

### Beispiel 2

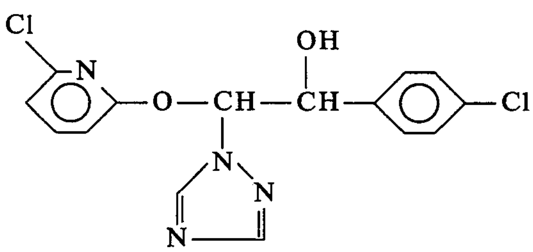

(Verfahren c)

8,7 g (0,025 Mol) 1-(6-Chlor-pyridin-2-yl-oxy)-2-(4-chlorphenyl)-1-(1,2,4-triazol-1-yl)-ethan-2-on (Beispiel 1) werden in 100 ml Methanol gelöst und mit 1 g (0,025 Mol) Natriumborhydrid versetzt. Man erhitzt 30 Minuten unter Rückfluß, engt durch Abdestillieren des Lösungsmittels im Vakuum ein und verteilt den Rückstand zwischen Chloroform/Wasser. Die organische Phase wird abgetrennt, nochmals mit Wasser ausgeschüttelt, über Magnesiumsulfat getrocknet und eingeengt. Man erhält 62 g (70% der Theorie) 1-(6-Chlor-pyridin-2-yl-oxy)-2-(4-chlorphenyl)-1-(1,2,4-triazol-1-yl)-ethan-2-ol als viskoses Öl.

Analog und gemäß den Verfahrensvarianten (a), (b) und (c) werden die folgenden Verbindungen der allgemeinen Formel (I)

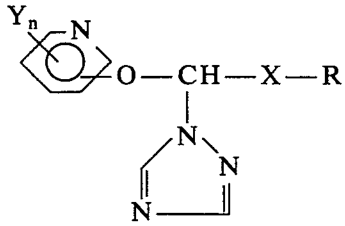

(I)

erhalten:

| Beispiel Nr. | 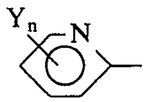 | X | R | Schmelzpunkt (°C) |
|---|---|---|---|---|
| 3 | 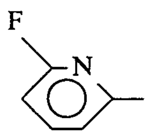 | CO | 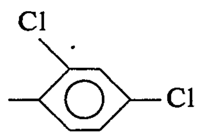 | 103 |
| 4 | 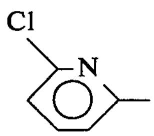 | CO | 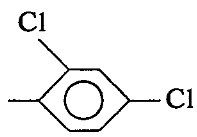 | 136 |
| 5 | 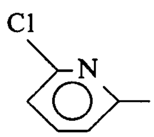 | CO | 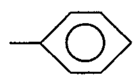 | 116 |
| 6 | 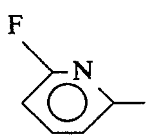 | CO | 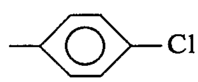 | 115 |
| 7 | 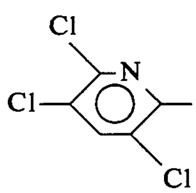 | CO | 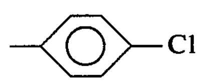 | 138 |
| 8 | 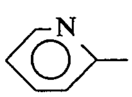 | CO | 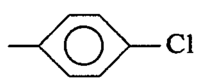 | 96 |
| 9 | 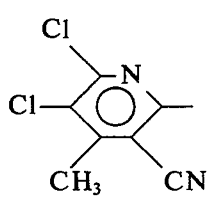 | CO | 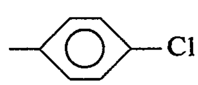 | 160 |
| 10 | 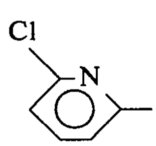 | CO | 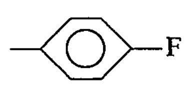 | 122 |

Fortsetzung

| Beispiel Nr. | 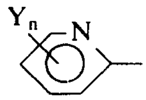 | X | R | Schmelzpunkt (°C) |
|---|---|---|---|---|
| 11 | 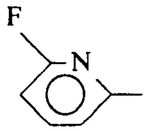 | CO | 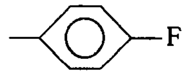 | 75 – 77 |
| 12 | 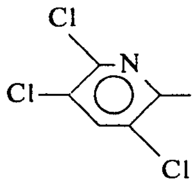 | CO | 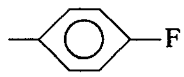 | 102 |
| 13 | 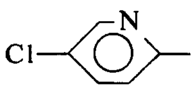 | CO | 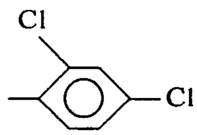 | 102 |
| 14 | 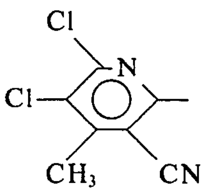 | CO | 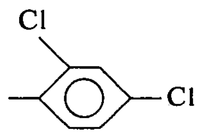 | 131 |
| 15 | 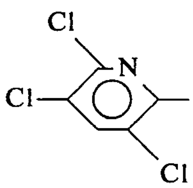 | CO | 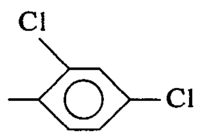 | 160 |
| 16 | 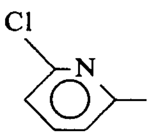 | CO | 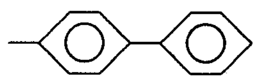 | 110 |
| 17 | 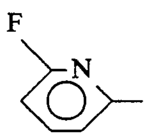 | CO | 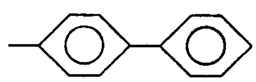 | 130 |
| 18 | 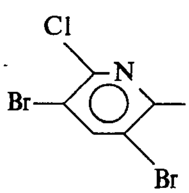 | CO | 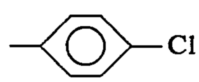 | 158 |
| 19 | 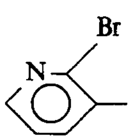 | CO | 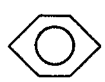 | 148 |

Fortsetzung

| Beispiel Nr. | 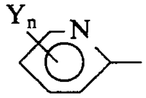 | X | R | Schmelzpunkt (°C) |
|---|---|---|---|---|
| 20 | 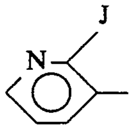 | CO | 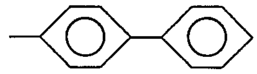 | 159 |
| 21 | 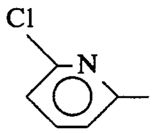 | CH(OH) | 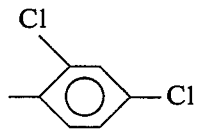 | Öl |
| 22 | 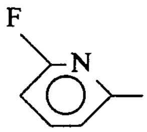 | CH(OH) | 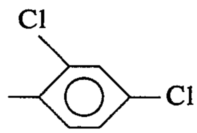 | Öl |
| 23 | 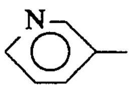 | CO | 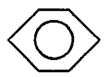 | Harz |
| 24 | 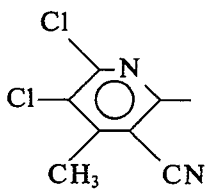 | CO | 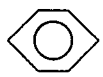 | 162 |
| 25 | 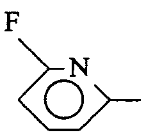 | CH(OH) | 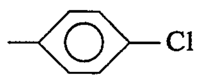 | 130 (A-Form)*) |
| 26 | 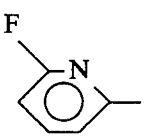 | CH(OH) | 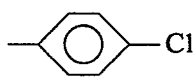 | 125 (B-Form)*) |
| 27 | 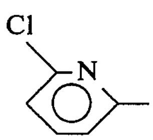 | CH(OH) | 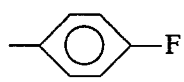 | 118 |
| 28 | 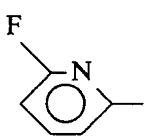 | CH(OH) | 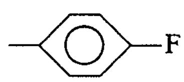 | 112 |
| 29 | 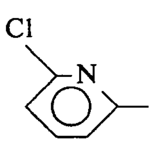 | CH(OH) | 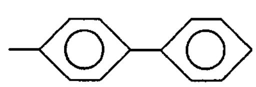 | 178 |

Fortsetzung

| Beispiel Nr. | 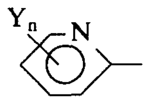 | X | R | Schmelzpunkt (°C) |
|---|---|---|---|---|
| 30 | 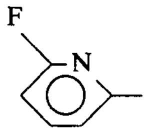 | CH(OH) | 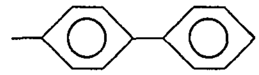 | 152 |

*) A- und B-Form = die beiden geometrischen Isomeren.

## Anwendungsbeispiele

In den nachfolgenden Beispielen werden die nachstehend angegebenen Verbindungen als Vergleichssubstanzen eingesetzt:

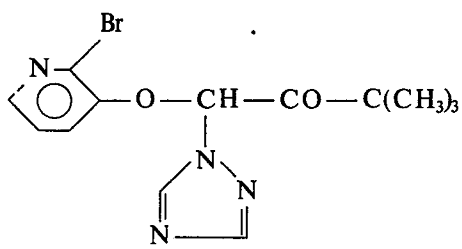

(A)

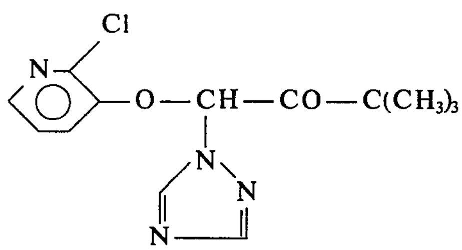

(B)

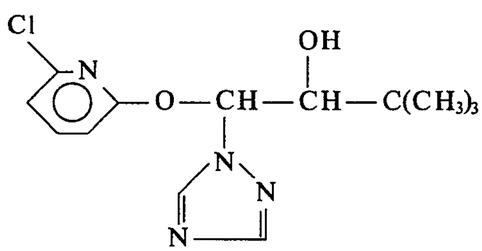

(C)

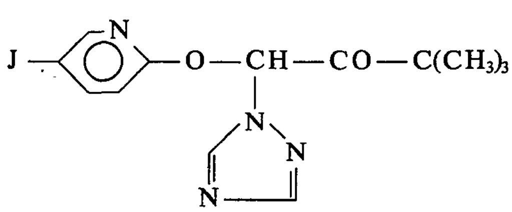

(D)

## Beispiel A

### Sproßbehandlungs-Test/Getreidemehltau/Protektiv (blattzerstörende Mykose)

Zur Herstellung einer zweckmäßigen Wirkstoffzubereitung nimmt man 0,25 Gewichtsteile Wirkstoff in Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 – 22° C und einer Luftfeuchtigkeit von 80 – 90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z. B. die folgenden Verbindungen eine sehr gute Wirkung, die derjenigen der aus dem Stand der Technik bekannten Verbindungen (A) und (B) überlegen ist:

Verbindungen gemäß Herstellungsbeispielen 3, 4, 1, 5 und 2.

## Beispiel B

### Gerstenmehltau-Test (Erysiphe graminis var. hordei)/systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21—22° C und 80—90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Bei diesem Test zeigen z. B. die folgenden Verbindungen eine sehr gute Wirkung, die derjenigen der aus dem Stand der Technik bekannten Verbindung (B) überlegen ist:

Verbindungen gemäß Herstellungsbeispielen: 3, 5 und 2.

## Beispiel C

### Fusioladium-Test (Apfel)/protektiv

Lösungsmittel: 4,7 Gewichtsteile Aceton
Emulgator: 0,3 Gewichtsteile Alkylarylpolyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20° C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindung (C) überlegen ist:
Verbindungen gemäß Herstellungsbeispiel 3, 4 und 19.

Beispiel D

Podosphaera-Test (Apfel)/protektiv

Lösungsmittel: 4,7 Gewichtsteile Aceton
Emulgator: 0,2 Gewichtsteile Alkyl-aryl-polyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 5-Blattstadium befinden, bis zur Tropfnässe. Die Pflanzen verbleiben 24 Stunden bei 20°C und einer relativen Luftfeuchtigkeit von 70% im Gewächshaus. Anschließend werden die durch Bestäuben mit Konidien des Apfelmehltauerregers (Podosphaera leucotricha) inokuliert und in ein Gewächshaus mit einer Temperatur von 21 bis 23° C und einer relativen Luftfeuchtigkeit von ca. 70° gebracht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindungen (A) und (D) überlegen ist:
Verbindungen gemäß Herstellungsbeispielen 3, 4, 5 und 2.

**Patentansprüche**

1. Triazoylphenacyl-pyridyl-ether-Derivate der Formel

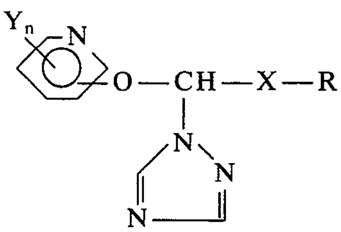

(I)

in welcher

R für Phenyl steht, welches gegebenenfalls substituiert sein kann durch Halogen, Alkyl und Alkoxy mit jeweils 1 bis 4 Kohlenstoffatomen, sowie durch gegebenenfalls durch Halogen substituiertes Phenyl und Phenoxy,
X für die Ketogruppe oder eine CH(OH)-Gruppierung steht,
Y für Halogen, Alkyl oder Alkoxy mit jeweils 1 bis 4 Kohlenstoffatomen oder für Cyano steht und
n für die Zahlen 0, 1, 2, 3 oder 4 steht,

und deren physiologisch verträglichen Säureadditions-Salze sowie Metallsalz-Komplexe.

2. Verfahren zur Herstellung von Triazolylphenacylpyridyl-ether-Derivaten der Formel

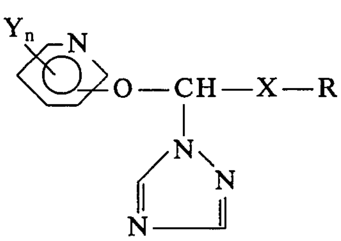

(I)

in welcher

R für Phenyl steht, welches gegebenenfalls substituiert sein kann durch Halogen, Alkyl und Alkoxy mit jeweils 1 bis 4 Kohlenstoffatomen, sowie durch gegebenenfalls durch Halogen substituiertes Phenyl und Phenoxy,

X für die Ketogruppe oder eine CH(OH)-Gruppierung steht,

Y für Halogen, Alkyl oder Alkoxy mit jeweils 1 bis 4 Kohlenstoffatomen oder für Cyano steht und

n für die Zahlen 0, 1, 2, 3 oder 4 steht,

dadurch gekennzeichnet, daß man

a) Triazolylhalogenketone der Formel

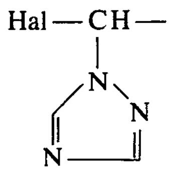

(II)

in welcher

R die oben angegebene Bedeutung hat und
Hal für Chlor oder Brom steht,

mit Pyridinolen der Formel

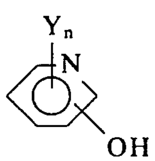

(III)

in welcher

Y und n die oben angegebene Bedeutung haben,

in Gegenwart eines Säurebindemittels und in Gegenwart eines Verdünnungsmittels umsetzt, oder

b) Halogenetherketone der Formel

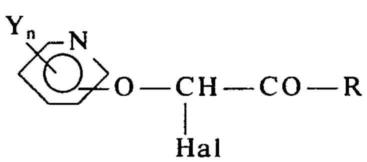

(IV)

in welcher

Hal, R, Y und n die oben angegebene Bedeutung haben,

mit 1,2,4-Triazol in Gegenwart eines Säurebindemittels und gegebenenfalls in Gegenwart eines Verdünnungsmittels umsetzt, und

c) gegebenenfalls die nach den Verfahrensvarianten (a) und (b) erhaltenen Keto-Derivate der Formel

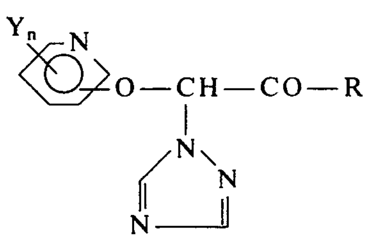

(Ia)

in welcher

R, Y und n die oben angegebene Bedeutung haben,

nach bekannten Methoden in üblicher Weise reduziert, und an die so erhaltenen Verbindungen der Formel (I) gegebenenfalls anschließend eine Säure oder ein Metallsalz addiert.

3. Fungizide Mittel, gekennzeichnet durch einen Gehalt an mindestens einem Triazolylphenacylpyridyl-ether-Derivat der Formel I in Ansprüchen 1 und 2.

4. Verwendung von Triazolylphenacyl-pyridyl-ether-Derivaten der Formel I in Ansprüchen 1 und 2 zur Bekämpfung von Pilzen.

## Claims

1. Triazolylphenacyl pyridyl ether derivatives of the formula

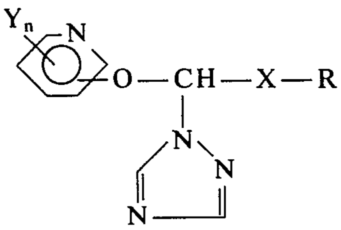

(I)

in which

R represents phenyl which can optionally be substituted by halogen, alkyl and alkoxy with in each case 1 to 4 carbon atoms, and by phenyl and phenoxy optionally substituted by halogen,
X represents the keto group or a CH(OH)-grouping,
Y represents halogen, alkyl or alkoxy with in each case 1 to 4 carbon atoms, or cyano and
n represents the number 0, 1, 2, 3 or 4,

and physiologically acceptable acid addition salts and metal salt complexes thereof.

2. Process for the preparation of triazolylphenacyl pyridyl ether derivatives of the formula

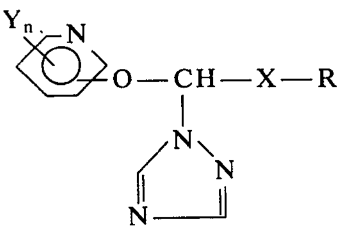

(I)

in which

R represents phenyl which can optionally be substituted by halogen, alkyl and alkoxy with in each case 1 to 4 carbon atoms, and by phenyl and phenoxy optionally substituted by halogen,
X represents the keto group or a CH(OH)-grouping,
Y represents halogen, alkyl or alkoxy with in each case 1 to 4 carbon atoms, or cyano and
n represents the number 0, 1, 2, 3 or 4,

characterised in that

a) triazolyhalogeno-ketones of the formula

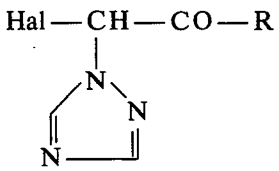

(II)

in which

R has the meaning indicated above and
Hal represents chlorine or bromine,

are reacted with pyridinols of the formula

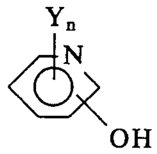

(III)

in which

Y and n have the meaning indicated above,

in the presence of an acid-binding agent and in the presence of a diluent, or

b) halogenoether-ketones of the formula

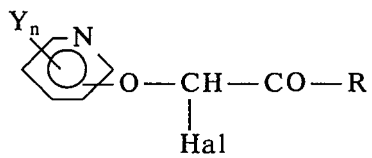

(IV)

in which

Hal, R, Y and n have the meaning indicated above,

are reacted with 1,2,4-triazole in the presence of an acid-binding agent and if appropriate in the presence of a diluent, and

c) if appropriate, the keto derivatives obtained according to process variants (a) and (b), of the formula

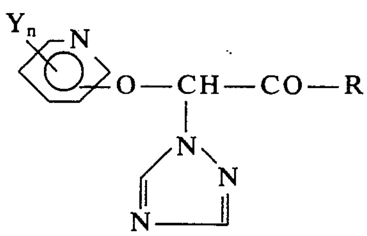

(Ia)

in which

R, Y and n have the meaning indicated above, are reduced by known methods in the customary manner, and, if appropriate, an acid or a metal salt is then added on to the compounds of the formula (I) thus obtained.

3. Fungicidal agents, characterised in that they contain at least one triazolylphenacyl pyridyl ether derivative of the formula I in claims 1 and 2.

4. Use of triazolylphenacyl pyridyl ether derivatives of the formula I in claims 1 and 2 for combating fungi.

**Revendications**

1. Derivés de traizolylphénacyle pyridyle éthers de formule:

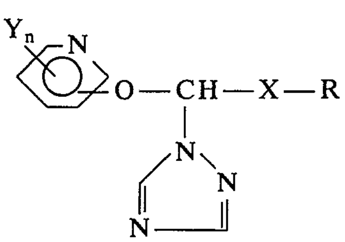

(I)

dans laquelle

R représente un groupe phényle qui peut le cas échéant être substitué par des halogènes, des groupes alkyle et alcoxy contenant chacun 1à 4 atomes de carbone, ou par un groupe phényle ou phénoxy éventuellement substitué par des halogènes.
X représente le groupe céto ou un groupe CH(OH),
Y représente un halogène, un groupe alkyle ou alcoxy contenant chacun 1 à 4 atomes de carbone ou le groupe cyano et,
n est égal à 0, 1, 2, 3 ou 4,

et leurs sels formés par addition avec des acides et complexes de sels métalliques tolérés par l'organisme.

2. Procédé de préparation des derivés de triazolylphénacyle pyridyle éthers de formule

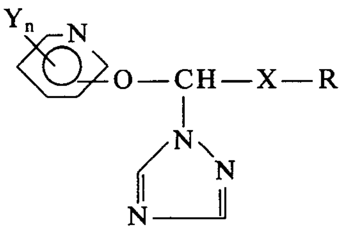

(I)

dans laquelle

R représente un groupe phényle qui peut éventuellement être substitué par des halogènes, des groupes alkyle et alcoxy contenant chacun 1 à 4 atomes de carbone, ou par un groupe phényle ou phénoxy éventuellement substitué par des halogènes,
X représente le groupe céto ou un groupe CH(OH),
Y représente un halogène, un groupe alkyle ou alcoxy contenant chacun 1 à 4 atomes de carbone ou le groupe cyano et,
n est égal à 0, 1, 2, 3 ou 4,

caractérisé en ce que:

a) on fait réagir des triazolylhalogénocétones de formule:

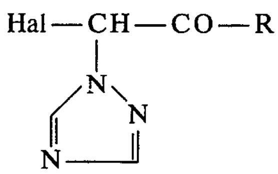

(II)

dans laquelle

R a les significations indiquées ci-dessus et
Hal représente le chlore ou le brome,

avec des pyridinols de formule:

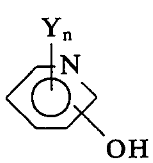

(III)

dans laquelle

Y et n ont les significations indiquées ci-dessus,

en présence d'un agent fixant les acides et en présence d'un diluant, ou bien

b) on fait réagir des halogéno-éther-cétones de formule:

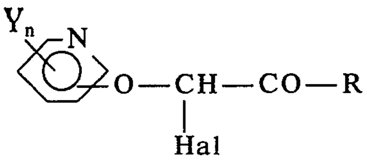

(IV)

dans laquelle

Hal, R, Y et n ont les significations indiquées ci-dessus,

avec le 1,2,4-triazole en présence d'un agent fixant les acides et le cas échéant en présence d'un diluant et,

c) le cas échéant, on réduit de la manière habituelle, selon des procédés connus, les dérivés cétoniques de formule

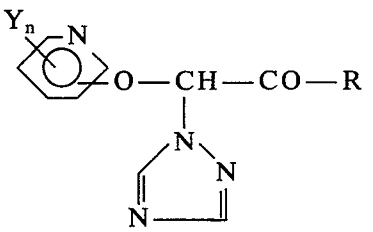

(Ia)

dans laquelle R, Y et n ont les significations indiquées ci-dessus, obtenus selon les variantes opératoires a) et b), et, sur les composés de formule I ainsi obtenus, on fixe le cas échéant par addition un acide ou un sel métallique.

3. Produit fongicide caractérisé en ce qu'il contient au moins un derivé de triazolylphénacyle pyridyle éthers de formule (I) des revendications 1 et 2.

4. Utilisation des derivés de triazolylphénacyle pyridyle éthers de formule (I) des revendications 1 et 2 dans la lutte contre les mycètes.